# FASCICULE DE BREVET EUROPEEN

(11) **EP 0 797 568 B1**
(45) Date de publication et mention de la délivrance du brevet: **31.10.2001**
(21) Numéro de dépôt: 95942242.9
(22) Date de dépôt: 12.12.1995
(51) Int. Cl.: C07C 255/24, C07C 253/30

(54) **PROCEDE D'HEMIHYDROGENATION DE DINITRILES EN AMINONITRILES**
VERFAHREN ZUR SELKTIVEN HYDRIERUNG VON DINITRILEN ZU AMINONITRILEN
METHOD FOR PARTIALLY HYDROGENATING DINITRILES TO AMINONITRILES

(30) Priorité: 14.12.1994 FR 9415282
(43) Date de publication de la demande: 01.10.1997
(73) Titulaire: RHODIA POLYAMIDE INTERMEDIATES, 69190 Saint-Fons (FR)
(72) Inventeur: COTTING, Marie-Christine, F-69500 Bron (FR); GILBERT, Laurent, F-75015 Paris (FR); LECONTE, Philippe, F-69330 Meyzieu (FR)
(74) Mandataire: Esson, Jean-Pierre
(86) Numéro de dépôt international: FR9501643
(87) Numéro de publication internationale: WO9618603

(56) Documents cités:
- WO-A-93/12073
- WO-A-93/16034
- US-A- 4 248 799
- US-A- 4 362 671
- US-A- 5 151 543

## Description

La présente invention concerne l'hémihydrogénation de dinitriles en aminonitriles correspondants.

Généralement l'hydrogénation des dinitriles est réalisée pour préparer les diamines correspondantes ; ainsi particulièrement l'hydrogénation de l'adiponitrile conduit à l'hexaméthylène diamine, elle-même l'un des deux composés de base de la préparation du polyamide-6,6.

Cependant,il peut parfois s'avérer nécessaire de préparer non la diamine, mais l'aminonitrile intermédiaire. C'est par exemple, mais non limitativement, le cas pour l'hémihydrogénation de l'adiponitrile en aminocapronitrile, composé susceptible d'être ensuite transformé en caprolactame composé de base du polyamide-6 ou directement en polyamide-6.

Ainsi le brevet US 4 389 348 décrit un procédé d'hydrogénation de dinitrile en oméga-aminonitrile, par l'hydrogène, en milieu solvant aprotique et ammoniac et en présence de rhodium déposé sur un support basique.

Le brevet US 5 151 543 décrit un procédé d'hydrogénation partielle de dinitriles en aminonitriles dans un solvant en excès molaire d'au moins 2/1 par rapport au dinitrile, comprenant de l'ammoniac liquide ou un alcanol contenant une base minérale soluble dans ledit alcanol, en présence d'un catalyseur de type nickel ou cobalt de Raney.

Le brevet WO-A-93/16034 décrit un procédé de préparation de 6-aminocapronitrile par hydrogénation de l'adiponitrile, sous pression et à une température de 50 à 90°C, en présence d'un hydroxyde de métal alcalin ou d'ammonium, d'un complexe d'un métal de transition à faible valence et d'un catalyseur nickel de Raney. La mise en oeuvre de ce procédé est effectuée selon les exemples dans un solvant tel qu'un alcanol inférieur ou un hydrocarbure.

La présente invention a trait à l'hydrogénation préférentielle d'une seule fonction nitrile d'un dinitrile (appelée dans le présent texte hémihydrogénation) de manière à préparer majoritairement l'aminonitrile correspondant et seulement de manière minoritaire la diamine.

Plus précisément l'invention concerne un procédé d'hémihydrogénation de dinitriles aliphatiques en aminonitriles correspondants, à l'aide d'hydrogène et en présence d'un catalyseur choisi parmi le nickel de Raney, le cobalt de Raney, le nickel de Raney comportant un élément dopant choisi parmi les éléments des groupes IVb, Vlb, Vllb et VIII de la classification périodique des éléments telle que publiée dans Handbook of Chemistry and Physics (Weast, 5ème édition de 1970-1971) et le zinc et le cobalt de Raney comportant un élément dopant choisi parmi les éléments des groupes IVb, Vlb, Vllb et VIII de la classification périodique des éléments et le zinc, et d'une base minérale forte dérivant d'un métal alcalin ou alcalino-terreux, caractérisé en ce que :
- le milieu initial d'hydrogénation comporte à l'absence de tout au solvant, de l'eau à raison d'au moins 0,5 % en poids par rapport à la totalité des composés liquides dudit milieu, de la diamine et de l'aminonitrile susceptibles de se former à partir du dinitrile à hydrogéner ainsi que du dinitrile non transformé à raison pour l'ensemble de ces trois composés de 80 % à 99,5 % en poids par rapport à la totalité des composés liquides dudit milieu,
- le taux de transformation du dinitrile peut atteindre 95 %
- et en ce que ledit procédé permet d'obtenir une sélectivité en aminonitriles visés d'au moins 60 %.

Le taux de transformation du dinitrile est de préférence d'au moins 70 %.

Les dinitriles aliphatiques qui peuvent être mis en oeuvre dans le procédé de l'invention sont plus particulièrement les dinitriles de formule générale (I) :

NC―R―CN (I)

dans laquelle R représente un groupement alkylène ou alcénylène, linéaire ou ramifié, ayant de 1 à 12 atomes de carbone.

De préférence, on met en oeuvre dans le procédé de l'invention des dinitriles de formule (I) dans laquelle R représente un radical alkylène, linéaire ou ramifié ayant de 1 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = (CH₂)₄ sera le plus fréquent car cela correspond à la mise en oeuvre de l'adiponitrile (ADN) dans le présent procédé.

La base minérale forte est généralement constituée par les hydroxydes, les carbonates et les alcanolates de métal alcalin ou de métal alcalino-terreux. Elle est choisie de préférence parmi les hydroxydes, les carbonates et les alcanolates de métal alcalin.

De façon privilégiée, la base minérale forte mise en oeuvre est choisie parmi les composés suivants : LiOH, NaOH, KOH, RbOH, CsOH et leur mélanges.

En pratique, on utilise le plus souvent NaOH et KOH, pour un bon compromis performance-prix, bien que RbOH et CsOH puissent donner de très bons résultats.

Le milieu réactionnel a une composition variant selon le type de mise en oeuvre du procédé.

En effet si le procédé est mise en oeuvre en mode discontinu comme c'est notamment le cas dans les réalisations de laboratoire ou de petites fabrications intermittentes, le milieu réactionnel initial s'enrichira progressivement en aminonitrile et, en moindre proportion, en diamine, tandis que la concentration en dinitrile pourra soit décroître si l'on charge la totalité ou la majeure partie dudit dinitrile dès le début de l'hémihydrogénation, soit demeurer relativement constante si le dinitrile est introduit progressivement au cours de la réaction.

Par contre, si le procédé est conduit en mode continu, la composition du milieu réactionnel en sortie de réacteur atteint des valeurs déterminées par les sélectivités de la réaction.

L'eau est habituellement présente dans une quantité inférieure ou égale à 20 %. Préférentiellement, la teneur en eau du milieu réactionnel est comprise entre 2 % et 15 % en poids par rapport à l'ensemble des constituants liquides dudit milieu.

La concentration de l'aminonitrile visé et/ou de la diamine correspondante et du dinitrile non transformé dans le milieu réactionnel est généralement comprise entre 85 % et 98 % en poids par rapport à l'ensemble des liquides inclus dans ledit milieu réactionnel.

En fonctionnement continu du procédé de l'invention, la composition en sortie de réacteur sera déterminée par le rapport des sélectivités respectives en aminonitrile et en diamine et par la vitesse d'introduction du dinitrile.

La quantité de base minérale forte est avantageusement supérieure ou égale à 0,1 mol/kg de catalyseur. De préférence, elle est comprise entre 0,1 mol et 3 mol par kg de catalyseur et plus préférentiellement encore entre 0,2 et 2 mol/kg de catalyseur.

Pour obtenir une sélectivité optimale en aminonitrile, le rapport base minérale forte/catalyseur peut être différencié selon la base mise en oeuvre. Ainsi avec KOH, RbOH et CsOH, ce rapport sera encore plus préférentiellement de 0,2 à 1,0 mol par kg de catalyseur, particulièrement de nickel de Raney dopé ou non. Avec NaOH et LiOH, ce rapport sera encore plus préférentiellement de 0,2 à 1,5 mol par kg de catalyseur, particulièrement de nickel de Raney dopé ou non.

Le catalyseur utilisé dans le procédé peut être un nickel de Raney, un cobalt de Raney, un nickel de Raney ou un cobalt de Raney comportant, outre le nickel ou le cobalt et les quantités résiduelles du métal éliminé de l'alliage d'origine lors de la préparation du catalyseur, c'est-à-dire généralement l'aluminium, un ou plusieurs autres éléments, souvent appelés dopants, tels que par exemple le chrome, le titane, le molybdène, le tungstène, le fer, le zinc. Parmi ces éléments dopants le chrome, le titane le fer et leurs mélanges sont considérés comme les plus avantageux. Ces dopants représentent habituellement, en poids par poids de nickel ou de cobalt, de 0 % à 15 % et de préférence de 0 % à 10 %.

Lorsque le catalyseur contient un dopant comme le chrome ou le titane, il est également avantageux de prendre en compte le rapport base forte/dopant. Ainsi on mettra en oeuvre de préférence un rapport KOH/dopant de 12 à 30 mol par kg de dopant et un rapport NaOH/dopant de 12 à 50 mol par kg de dopant.

La quantité de catalyseur mise en oeuvre peut varier très largement en fonction notamment du mode de fonctionnement adopté ou des conditions réactionnelles choisies. Ainsi, si l'on introduit progressivement le dinitrile dans le milieu réactionnel, le rapport pondéral catalyseur/dinitrile à hydrogéner sera beaucoup plus élevé que si tout le dinitrile est mis en oeuvre dès le début de la réaction. A titre indicatif, on peut utiliser de 0,5 % à 50 % en poids de catalyseur par rapport au poids total du milieu réactionnel et le plus souvent de 1 % à 35 % .

Pour un catalyseur donné et pour un taux de transformation donné du dinitrile, le rendement en aminonitrile passe par un maximum déterminé par le rapport base/Ni ou base/Co choisi dans les zones de valeurs indiquées précédemment.

La sélectivité globale en aminonitrile est améliorée par l'augmentation de la valeur de la constante de vitesse d'hydrogénation du dinitrile en aminonitrile et non par la diminution de la valeur de la constante de vitesse de l'hydrogénation de l'aminonitrile en diamine. C'est essentiellement sur la constante de vitesse de la première des deux réactions consécutives que jouent les différents paramètres indiqués précédemment.

Le procédé de l'invention est généralement mis en oeuvre à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 1bar (0,10 MPa) et 100 bar (10 MPa) et de préférence entre 5 bar (0,5 MPa) et 50 bar (5 MPa).

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui constitue le mode industriel préférable pour le procédé selon l'invention, la durée n'est évidemment pas un paramètre figeable.

Il est à noter que l'homme du métier peut moduler la chronologie des étapes du procédé selon l'invention, selon les conditions opératoires.

Les autres conditions qui régissent l'hydrogénation (en mode continu ou discontinu) conforme à l'invention, relèvent de dispositions techniques traditionnelles et connues en elles-mêmes.

Les exemples qui suivent illustrent l'invention. Dans ces exemples les abréviations suivantes pourront être utilisées :
- ADN = adiponitrile
- ACN = aminocapronitrile
- HMD = hexaméthylène diamine
- TT = taux de transformation
- RT = sélectivité par rapport au substrat de départ transformé (ici par rapport à l'ADN).

### EXEMPLE 1

Dans un réacteur en acier inoxydable de 300 ml, équipé d'une agitation de type rushtone cavitator, de moyens d'introduction des réactifs et de l'hydrogène et d'un système de régulation de température, on charge :
- adiponitrile 95,1 g
- hexaméthylène diamine 94,2 g
- eau 21,1 g
- KOH 0,056 g
- Ni de Raney (à 1,7 % de Cr) 2,5 g

Dans cet exemple il y a 0,4 mol KOH/kg Ni de Raney.

On chauffe le mélange réactionnel à 50°C après avoir purgé le réacteur à l'azote, puis à l'hydrogène ; la pression est alors réglée à 2 MPa à cette température par addition continue d'hydrogène. L'avancement de la réaction est suivi par la consommation d'hydrogène et l'analyse par chromatographie en phase vapeur (CPG) d'un prélèvement du mélange réactionnel. Lorsque l'optimum de rendement est atteint, on arrête la réaction par arrêt de l'agitation et refroidisssement du mélange réactionnel.

On obtient les résultats suivants :
- durée de la réaction : 80 min
- TT de l'ADN : 83,5 %
- RT en ACN : 77,5 %

### EXEMPLE 2

On répète l'exemple 1 dans les mêmes conditions opératoires avec les charges suivantes :
- adiponitrile 95,1 g
- hexaméthylène diamine 95,4 g
- eau 21,1 g
- KOH 0,113 g
- Ni de Raney (à 1,7 % de Cr) 2,5 g

Dans cet exemple il y a 0,8 mol KOH/kg Ni de Raney.

On obtient les résultats suivants :
- durée de la réaction : 80 min
- TT de l'ADN : 81,9%
- RT en ACN : 68,3 %

### EXEMPLE 3

On répète l'exemple 1 dans les mêmes conditions opératoires avec les charges suivantes :
- adiponitrile 27,4 g
- hexaméthylène diamine 166,4 g
- eau 19,4 g
- KOH 0,115 g
- Ni de Raney (à 1,7 % de Cr) 2,5 g

Dans cet exemple il y a 0,8 mol KOH/kg Ni de Raney.

On obtient les résultats suivants :
- durée de la réaction : 20 min
- TT de l'ADN : 71,2 %
- RT en ACN : 77,0 %

### EXEMPLE 4

On répète l'exemple 1 dans les mêmes conditions opératoires avec les charges suivantes :
- adiponitrile 142,4 g
- hexaméthylène diamine 47,75 g
- eau 21,1 g
- KOH 0,053 g
- Ni de Raney (à 1,7 % de Cr) 2,5 g

Dans cet exemple il y a 0,4 mol KOH/kg Ni de Raney.

On obtient les résultats suivants :
- durée de la réaction : 103 min
- TT de l'ADN: 76,2 %
- RT en ACN : 77,2 %

### EXEMPLE 5

On répète l'exemple 1 dans les mêmes conditions opératoires avec les charges suivantes :
- adiponitrile 95,1 g
- hexaméthylène diamine 97,1 g
- eau 21,1 g
- KOH 0,056 g
- Ni de Raney (à 2,4 % de Cr et 1,3 % de Fe) 2,5 g

Dans cet exemple il y a 0,4 mol KOH/kg Ni de Raney et 16,7 mol KOH/kg Cr.

On obtient les résultats suivants :
- durée de la réaction : 45 min
- TT de l'ADN : 82,4 %
- RT en ACN : 74,3 %

### EXEMPLE 6

On répète l'exemple 1 dans les mêmes conditions opératoires avec les charges suivantes :
- adiponitrile 95,0 9
- hexaméthylène diamine 97,3 g
- eau 21,1 g
- KOH 0,056 g
- Ni de Raney (à 3,0 % de Cr et 1,6 % de Fe) 2,5 g

Dans cet exemple il y a 0,4 mol KOH/kg Ni de Raney et 13,3 mol KOH/kg Cr.

On obtient les résultats suivants :
- durée de la réaction : 85 min
- TT de l'ADN : 84,5 %
- RT en ACN : 65,9 %

### EXEMPLE 7

Dans un réacteur en acier inoxydable de 150 ml, équipé d'une agitation magnétique, de moyens d'introduction des réactifs et de l'hydrogène et d'un système de régulation de température, on charge :
- adiponitrile 21,65 g
- hexaméthylène diamine 21,65 g
- eau 4,75 g
- NaOH 0,0372 g
- Ni de Raney (à 1,7 % de Cr) 0,58 g

Dans cet exemple il y a 1,6 mol NaOH/kg Ni de Raney.

On chauffe le mélange réactionnel à 50°C après avoir purgé le réacteur à l'azote, puis à l'hydrogène ; la pression est alors réglée à 2 MPa à cette température par addition continue d'hydrogène. L'avancement de la réaction est suivi par la consommation d'hydrogène et l'analyse par chromatographie en phase vapeur (CPG) d'un prélèvement du mélange réactionnel. Lorsque l'optimum de rendement est atteint, on arrête la réaction par arrêt de l'agitation et refroidisssement du mélange réactionnel.

On obtient les résultats suivants :
- durée de la réaction : 90 min
- TT de l'ADN : 70 %
- RT en ACN: 62%

### EXEMPLE 8

On répète l'exemple 7 dans les mêmes conditions opératoires avec les charges suivantes :
- adiponitrile 21,6 g
- hexaméthylène diamine 21,8 g
- eau 4,75 g
- NaOH 0,0046 g
- Ni de Raney (à 1,7 % de Cr) 0,58 g

Dans cet exemple il y a 0,2 mol NaOH/kg Ni de Raney.

On obtient les résultats suivants :
- durée de la réaction : 107 min
- TT de l'ADN : 76 %
- RT en ACN : 62 %

### EXEMPLE 9

On répète l'exemple 7 dans les mêmes conditions opératoires avec les charges suivantes :
- adiponitrile 21,6 g
- hexaméthylène diamine 21,7 g
- eau 4,78 g
- NaOH 0,0094 g
- Ni de Raney (à 1,7 % de Cr) 0,58 g

Dans cet exemple il y a 0,4 mol NaOH/kg Ni de Raney.

On obtient les résultats suivants :
- durée de la réaction : 67 min
- TT de l'ADN : 80 %
- RT en ACN : 69%

### EXEMPLE 10

On répète l'exemple 7 dans les mêmes conditions opératoires avec les charges suivantes :
- adiponitrile 21,6 g
- hexaméthylène diamine 21,6 g
- eau 4,75 g
- NaOH 0,0187 g
- Ni de Raney (à 1,7 % de Cr) 0,58 g

Dans cet exemple il y a 0,8 mol NaOH/kg Ni de Raney.

On obtient les résultats suivants :
- durée de la réaction : 69 min
- TT de l'ADN : 75%
- RT en ACN : 73 %

### EXEMPLE 11

On répète l'exemple 7 dans les mêmes conditions opératoires avec les charges suivantes :
- adiponitrile 21,65 g
- hexaméthylène diamine 21,65 g
- eau 4,75 g
- KOH 0,026 g
- Ni de Raney (à 3,6 % de Cr) 0,58 g

Dans cet exemple il y a 0,8 mol KOH/kg Ni de Raney.

On obtient les résultats suivants :
- durée de la réaction : 51 min
- TT de l'ADN : 75 %
- RT en ACN : 67 %

### EXEMPLE 12

Dans un réacteur métallique, équipé d'une agitation de type cavitator, de moyens d'introduction des réactifs et de l'hydrogène et de différents systèmes de régulation, on introduit avec les charges suivantes :
- adiponitrile 2856 kg
- hexaméthylène diamine 1151 kg
- eau 588 kg
- KOH 0,83 kg
- Ni de Raney (à 1,7 % de Cr) 37 kg

On opère dans les conditions décrites pour l'exemple 1.

On obtient les résultats suivants :
- durée de la réaction : 3 h 30
- TT de l'ADN : 86 %
- RT en ACN : 64 %

### EXEMPLE 13

Dans un réacteur en acier inoxydable de 150 ml, équipé d'une agitation magnétique, de moyens d'introduction des réactifs et de l'hydrogène et d'un système de régulation de température, on charge :
- adiponitrile 6 g
- hexaméthylène diamine 41,16 g
- eau 0,84 g
- CsOH 0,054 g
- Ni de Raney (à 12 % de Fe) 0,4 g

Dans cet exemple il y a 0,9 mol CsOH/kg Ni de Raney.

On chauffe le mélange réactionnel à 80°C après avoir purgé le réacteur à l'azote, puis à l'hydrogène ; la pression est alors réglée à 2,5 MPa à cette température par addition continue d'hydrogène. L'avancement de la réaction est suivi par la consommation d'hydrogène et l'analyse par chromatographie en phase vapeur (CPG) d'un prélèvement du mélange réactionnel. Lorsque l'optimum de rendement est atteint, on arrête la réaction par arrêt de l'agitation et refroidisssement du mélange réactionnel.

On obtient les résultats suivants :
- durée de la réaction : 50 min
- TT de l'ADN : 89 %
- RT en ACN : 65 %

### EXEMPLE 14

On répète l'exemple 13 dans les mêmes conditions opératoires avec les charges suivantes :
- adiponitrile 6 g
- hexaméthylène diamine 37,8 g
- eau 4,2 g
- CsOH 0,036 g
- Ni de Raney (à 1,5 % de Ti) 0,4 g

Dans cet exemple il y a 0,6 mol CsOH/kg Ni de Raney.

On obtient les résultats suivants :
- durée de la réaction : 20 min
- TT de l'ADN : 90 %
- RT en ACN : 60 %

## Revendications

1. Procédé d'hémihydrogénation de dinitriles en aminonitriles correspondants, à l'aide d'hydrogène et en présence d'un catalyseur choisi parmi le nickel de Raney, le cobalt de Raney, le nickel de Raney comportant un élément dopant choisi parmi les éléments des groupes IVb, Vlb, VIIb et VIII de la classification périodique des éléments et le zinc et le cobalt de Raney comportant un élément dopant choisi parmi les éléments des groupes IVb, Vlb, Vllb et VIII de la classification périodique des éléments et le zinc, et d'une base minérale forte dérivant d'un métal alcalin ou alcalino-terreux, **caractérisé en ce que** :
- le milieu initial d'hydrogénation comporte, en l'absence de tout autre solvant, de l'eau à raison d'au moins 0,5 % en poids et d'au plus 20 % par rapport à la totalité des composés liquides dudit milieu, de la diamine et de l'aminonitrile susceptibles de se former à partir du dinitrile à hydrogéner ainsi que du dinitrile non transformé à raison pour l'ensemble de ces trois composés de 80 % à 99,5 % en poids par rapport à la totalité des composés liquides dudit milieu.

2. Procédé selon la revendication 1, **caractérisé en ce que** la base minérale est choisie parmi les hydroxydes, les carbonates et les alcanolates de métal alcalin ou de métal alcalino-terreux et de préférence parmi les hydroxydes, les carbonates et les alcanolates de métal alcalin.

3. Procédé selon l'une des revendications 1 à 2, **caractérisé en ce que** la base minérale forte mise en oeuvre est choisie parmi les composés suivants : LiOH, NaOH, KOH, RbOH, CsOH et leur mélanges.

4. Procédé selon l'une des revendications 1 à 3, **caractérisé en ce que** la quantité de base minérale présente dans le milieu réactionnel est supérieure ou égale à 0,1 mole par kilogramme de catalyseur.

5. Procédé selon la revendication 4, **caractérisé en ce que** la quantité de base minérale présente dans le milieu réactionnel est comprise entre 0,1 et 3 moles par kilogramme de catalyseur

6. Procédé selon l'une des revendications 1 à 5, **caractérisé en ce que** la quantité de base minérale KOH, RbOH, CsOH présente dans le milieu réactionnel est de 0,2 à 1,0 mol par kg de catalyseur.

7. Procédé selon l'une des revendications 1 à 5, **caractérisé en ce que** la quantité de base minérale NaOH, LiOH présente dans le milieu réactionnel est de 0,2 à 1,5 mol par kg de catalyseur.

8. Procédé selon la revendication 6 ou 7, **caractérisé en ce que** le catalyseur est du nickel de Rany dopé ou non.

9. Procédé selon l'une des revendications 1 à 8, **caractérisé en ce que** les dinitriles aliphatiques qui peuvent être mis en oeuvre dans le procédé de l'invention sont les dinitriles de formule générale (I):
NC-R-CN (I)
dans laquelle R représente un groupement alkylène ou alcénylène, linéaire ou ramifié, ayant de 1 à 12 atomes de carbone et de préférence R représente un radical alkylène, linéaire ou ramifié ayant de 1 à 6 atomes de carbone.

10. Procédé selon l'une des revendications 1 à 9, **caractérisé en ce que** l'eau est présente dans le milieu réactionnel dans une quantité comprise entre 2 % et 15 % en poids par rapport à l'ensemble des constituants liquides dudit milieu.

11. Procédé selon l'une des revendications 1 à 10, **caractérisé en ce que** la concentration de l'aminonitrile visé et/ou de la diamine correspondante et du dinitrile non transformé dans le milieu réactionnel est comprise entre 85 % et 98 % en poids par rapport à l'ensemble des liquides inclus dans ledit milieu réactionnel.

12. Procédé selon l'une des revendications 1 à 7 et 9 à 11, **caractérisé en ce que** le catalyseur utilisé est choisi parmi un nickel de Raney, un cobalt de Raney, un nickel de Raney ou un cobalt de Raney comportant un ou plusieurs autres éléments dopants, tels que le chrome, le titane, le molybdène, le tungstène, le fer, le zinc.

13. Procédé selon l'une des revendications 1 à 12, **caractérisé en ce que** le catalyseur utilisé est choisi parmi un nickel de Raney comportant au moins un élément dopant choisi parmi le chrome, le titane, le fer et leurs mélanges.

14. Procédé selon l'une des revendications 1 à 13, **caractérisé en ce que** le catalyseur utilisé est choisi parmi un nickel de Raney comportant de 0 % à 15 % d'au moins un élément dopant en poids par poids de nickel.

15. Procédé selon l'une des revendications 1 à 14, **caractérisé en ce que** le catalyseur contient un dopant choisi parmi le chrome ou le titane et que l'on met en oeuvre rapport KOH/dopant de 12 à 30 mol par kg de dopant ou un rapport NaOH/dopant de 12 à 50 mol par kg de dopant.

16. Procédé selon l'une des revendications 1 à 15, **caractérisé en ce que** le catalyseur mis en oeuvre représente de 0,5 % à 50 % en poids par rapport au poids total du milieu réactionnel.

17. Procédé selon l'une des revendications 1 à 14, **caractérisé en ce qu'**il est mis en oeuvre à une température réactionnelle inférieure ou égale à 150°C.

18. Procédé selon la revendication 17, **caractérisé en ce que** la température réactionnelle est inférieure ou égale à 100°C.

19. Procédé selon l'une des revendications 1 à 18, **caractérisé en ce que** l'on opère à une pression en hydrogène comprise entre 1bar (0,10 MPa) et 100 bar (10 MPa).

## Patentansprüche

1. Verfahren zur Hemihydrierung von Nitrilen zu den entsprechenden Aminonitrilen mit Hilfe von Wasserstoff in Gegenwart eines Katalysators, ausgewählt unter Raney-Nickel, Raney-Kobalt, Raney-Nickel, umfassend ein Dotierungselement, ausgewählt unter den Elementen der Gruppen IVb, VIb, VIIb und VIII des Periodensystems der Elemente und Zink,und Raney-Kobalt, umfassend ein Dotierungselement, ausgewählt unter den Elementen der Gruppen IVb, VIb, VIIb und VIII des Periodensystems der Elemente und Zink,und einer starken Mineralbase, abgeleitet von einem Alkali- oder Erdalkalimetall, **dadurch gekennzeichnet, daß**
- das anfängliche Hydrierungsmilieu in Abwesenheit von jeglichem weiteren Lösungsmittel Wasser in einer Menge von wenigstens 0,5 Gew.% und höchstens 20 Gew.% in Bezug auf die Gesamtheit der flüssigen Verbindungen des besagten Milieus, Diamin und Aminonitril, die sich aus dem zu hydrierenden Dinitril bilden können, sowie nicht umgewandeltes Dinitril in einer Menge im Hinblick auf die Gesamtheit dieser drei Verbindungen von 80 bis 99 Gew.%, bezogen auf die Gesamtheit der flüssigen Verbindungen des besagten Milieus, umfaßt.

2. Verfahren gemäß Anspruch 1, **dadurch gekennzeichnet, daß** die mineralische Base unter den Hydroxiden, den Carbonaten, den Alkanolaten von Alkalimetall oder Erdalkalimetall, und vorzugsweise unter den Hydroxiden, den Carbonaten und den Alkanolaten von Alkalimetall ausgewählt wird.

3. Verfahren gemäß einem der Ansprüche 1 bis 2, **dadurch gekennzeichnet, daß** die eingesetzte starke mineralische Base unter den folgenden Verbindungen ausgewählt wird: LiOH, NaOH, KOH, RbOH, CsOH und deren Gemischen.

4. Verfahren gemäß einem der Ansprüche 1 bis 3, **dadurch gekennzeichnet, daß** die Menge der in dem Reaktionsmilieu anwesenden mineralischen Base größer ist als oder gleich 0,1 Mol je kg Katalysator.

5. Verfahren gemäß Anspruch 4, **dadurch gekennzeichnet, daß** die Menge der in dem Reaktionsmilieu vorhandenen mineralischen Base zwischen 0,1 und 3 Mol je kg Katalysator liegt.

6. Verfahren gemäß einem der Ansprüche 1 bis 5, **dadurch gekennzeichnet, daß** die Menge der in dem Reaktionsmilieu vorhandenen mineralischen Base KOH, RbOH, CsOH 0,2 bis 1,0 Mol je kg Katalysator beträgt.

7. Verfahren gemäß einem der Ansprüche 1 bis 5, **dadurch gekennzeichnet, daß** die Menge der in dem Reaktionsmilieu vorhandenen mineralischen Base NaOH, LiOH 0,2 bis 1,5 Mol je kg Katalysator beträgt.

8. Verfahren gemäß Anspruch 6 oder 7, **dadurch gekennzeichnet, daß** der Katalysator dotiertes oder nicht dotiertes Raney-Nickel ist.

9. Verfahren gemäß einem der Ansprüche 1 bis 8, **dadurch gekennzeichnet, daß** die aliphatischen Dinitrile, die bei dem erfindungsgemäßen Verfahren eingesetzt werden können, Dinitrile der allgemeinen Formel (I) sind:
NC-R-CN (I)
worin R eine lineare oder verzweigte Alkylen- oder Alkenylengruppe mit 1 bis 12 Kohlenstoffatomen bedeutet und vorzugsweise R einen linearen oder verzweigten Alkylenrest mit 1 bis 6 Kohlenstoffatomen bedeutet.

10. Verfahren gemäß einem der Ansprüche 1 bis 9, **dadurch gekennzeichnet, daß** Wasser in dem Reaktionsmilieu in einer Menge zwischen 2 und 15 Gew.%, bezogen auf die Gesamtheit der flüssigen Bestandteile des besagten Milieus, vorhanden ist.

11. Verfahren gemäß einem der Ansprüche 1 bis 10, **dadurch gekennzeichnet, daß** die Konzentration des in Betracht gezogenen Aminonitrils und/oder des entsprechenden Diamins und des nicht umgewandelten Dinitrils in dem Reaktionsmilieu zwischen 85 und 98 Gew.%, bezogen auf die Gesamtheit der in besagtem Reaktionsmilieu enthaltenen Flüssigkeiten, umfaßt.

12. Verfahren gemäß einem der Ansprüche 1 bis 7 und 9 bis 11, **dadurch gekennzeichnet, daß** der verwendete Katalysator unter einem Raney-Nickel, einem Raney-Kobalt, einem Raney-Nickel oder einem Raney-Kobalt, umfassend eines oder mehrere Dotierungselemente wie Chrom, Titan, Molybdän, Wolfram, Eisen, Zink, ausgewählt wird.

13. Verfahren gemäß einem der Ansprüche 1 bis 12, **dadurch gekennzeichnet, daß** der verwendete Katalysator unter einem Raney-Nickel ausgewählt wird, das zumindest ein Dotierungselement, ausgewählt unter Chrom, Titan, Eisen und deren Gemischen, umfaßt.

14. Verfahren gemäß einem der Ansprüche 1 bis 13, **dadurch gekennzeichnet, daß** der verwendete Katalysator unter einem Raney-Nickel ausgewählt wird, das 0 bis 15 Gew.% zumindest eines Dotierungselements, bezogen auf das Gewicht des Nickels, umfaßt.

15. Verfahren gemäß einem der Ansprüche 1 bis 14, **dadurch gekennzeichnet, daß** der Katalysator ein Dotierungsmittel, ausgewählt unter Chrom oder Titan, enthält und daß man ein Verhältnis KOH/Dotierungsmittel von 12 bis 30 Mol je kg Dotierungsmittel oder ein Verhältnis NaOH/Dotierungsmittel von 12 bis 50 Mol je kg Dotierungsmittel, anwendet.

16. Verfahren gemäß einem der Ansprüche 1 bis 15, **dadurch gekennzeichnet, daß** der eingesetzte Katalysator 0,5 bis 50 Gew.%, bezogen auf das Gesamtgewicht des Reaktionsmilieus, ausmacht.

17. Verfahren gemäß einem der Ansprüche 1 bis 14, **dadurch gekennzeichnet, daß** man eine Reaktionstemperatur von geringer als oder gleich 150°C anwendet.

18. Verfahren gemäß Anspruch 17, **dadurch gekennzeichnet, daß** die Reaktionstemperatur weniger als oder gleich 100°C beträgt.

19. Verfahren gemäß einem der Ansprüche 1 bis 18, **dadurch gekennzeichnet, daß** man bei einem Wasserstoffdruck zwischen 1 bar (0,10 MPa) und 100 bar (10 MPa) arbeitet.

## Claims

1. Process for the partial hydrogenation of dinitriles to the corresponding aminonitriles, with the aid of hydrogen and in the presence of a catalyst chosen from Raney nickel, Raney cobalt, Raney nickel comprising a doping element chosen from the elements of groups IVb, VIb, VIIb and VIII of the Periodic Classification of the elements and zinc and Raney cobalt comprising a doping element chosen from the elements of groups IVb, VIb, VIIb and VIII of the Periodic Classification of the elements and zinc, and of a strong inorganic base derived from an alkali or alkaline-earth metal, **characterized in that**:
- the initial hydrogenation mixture comprises, in the absence of any other solvent, water in a proportion of at least 0.5 % by weight and at most 20% relative to the totality of the liquid compounds of the said mixture, the diamine and the aminonitrile which are liable to be formed from the dinitrile to be hydrogenated and the unconverted dinitrile in a proportion of 80 % to 99.5 % by weight relative to the totality of liquid compounds of the said mixture, for the combined total of these three compounds.

2. Process according to Claim 1, **characterized in that** the inorganic base is chosen from alkali metal or alkaline-earth metal hydroxides, carbonates and alkanolates and preferably from alkali metal hydroxides, carbonates and alkanolates.

3. Process according to either of Claims 1 and 2, **characterized in that** the strong inorganic base used is chosen from the following compounds: LiOH, NaOH, KOH, RbOH, CsOH and mixtures thereof.

4. Process according to one of Claims 1 to 3, **characterized in that** the quantity of inorganic base which is present in the reaction mixture is greater than or equal to 0.1 mole per kilogram of catalyst.

5. Process according to Claim 4, **characterized in that** the quantity of inorganic base which is present in the reaction mixture is between 0.1 and 3 moles per kilogram of catalyst.

6. Process according to one of Claims 1 to 5, **characterized in that** the quantity of KOH, RbOH or CsOH inorganic base present in the reaction mixture is from 0.2 to 1.0 mol per kg of catalyst.

7. Process according to one of Claims 1 to 5, **characterized in that** the quantity of NaOH or LiOH inorganic base present in the reaction mixture is from 0.2 to 1.5 mol per kg of catalyst.

8. Process according to Claim 6 or 7, **characterized in that** the catalyst is Raney nickel, whether doped or not.

9. Process according to one of Claims 1 to 8, **characterized in that** the aliphatic dinitriles which may be used in the process of the invention are the dinitriles of general formula (I):
NC-R-CN (I)
in which R denotes a linear or branched alkylene or alkenylene group containing from 1 to 12 carbon atoms and preferably R denotes a linear or branched alkylene radical containing from 1 to 6 carbon atoms.

10. Process according to one of Claims 1 to 9, **characterized in that** water is present in the reaction mixture in a quantity of between 2 % and 15 % by weight relative to the combined total of the liquid constituents of the said mixture.

11. Process according to one of Claims 1 to 10, **characterized in that** the concentration of the aminonitrile aimed at and/or of the corresponding diamine and of the unconverted dinitrile in the reaction mixture is between 85 % and 98 % by weight relative to the combined total of the liquids included in the said reaction mixture.

12. Process according to one of Claims 1 to 7 and 9 to 11, **characterized in that** the catalyst employed is chosen from a Raney nickel, a Raney cobalt, a Raney nickel or a Raney cobalt comprising one or several other doping elements such as chromium, titanium, molybdenum, tungsten, iron or zinc.

13. Process according to one of Claims 1 to 12, **characterized in that** the catalyst employed is chosen from a Raney nickel comprising at least one doping element chosen from chromium, titanium, iron and their mixtures.

14. Process according to one of Claims 1 to 13, **characterized in that** the catalyst employed is chosen from a Raney nickel comprising from 0 % to 15 % of at least one doping element by weight per weight of nickel.

15. Process according to one of Claims 1 to 14, **characterized in that** the catalyst contains a dopant chosen from chromium or titanium and that a KOH/dopant ratio of 12 to 30 mol per kg of dopant or an NaOH/dopant ratio of 12 to 50 mol per kg of dopant is used.

16. Process according to one of Claims 1 to 15, **characterized in that** the catalyst used represents from 0.5 % to 50 % by weight relative to the total weight of the reaction mixture.

17. Process according to one of Claims 1 to 14, **characterized in that** it is carried out at a reaction temperature which is lower than or equal to 150°C.

18. Process according to Claim 17, **characterized in that** the reaction temperature is lower than or equal to 100°C.

19. Process according to one of Claims 1 to 18, **characterized in that** the operation is carried out at a hydrogen pressure of between 1 bar (0.10 MPa) and 100 bar (10 MPa) .
